# EUROPÄISCHE PATENTSCHRIFT

(11) **EP 4 622 693 B1**
(45) Veröffentlichungstag und Bekanntmachung des Hinweises auf die Patenterteilung: **29.04.2026**
(21) Anmeldenummer: 23822240.0
(22) Anmeldetag: 23.11.2023
(51) Int. Cl.: A61M 11/00, A61M 13/00, A61M 31/00, B05B 1/14, B05B 1/28

(54) **MEDIZINISCHES INSTRUMENT ZUM MULTIDIREKTIONALEN VERDÜSEN EINES FLUIDS IN EINEN HOHLRAUM EINES KÖRPERS UND WERKZEUG DAFÜR**
MEDICAL INSTRUMENT FOR THE MULTIDIRECTIONAL ATOMISING OF A FLUID INTO A CAVITY OF A BODY, AND TOOL THEREFOR
INSTRUMENT MÉDICAL POUR L'ATOMISATION MULTIDIRECTIONNELLE D'UN FLUIDE DANS UNE CAVITÉ D'UN CORPS, ET OUTIL ASSOCIÉ

(30) Priorität: 24.11.2022 DE 102022131155
(43) Veröffentlichungstag der Anmeldung: 01.10.2025
(73) Patentinhaber: Reger Medizintechnik GmbH, 78667 Villingendorf (DE)
(72) Erfinder: HETZEL, Alexander, 78667 Villingendorf (DE)
(74) Vertreter: mepat Patentanwälte
(86) Internationale Anmeldenummer: PCT/EP2023/082840
(87) Internationale Veröffentlichungsnummer: WO 2024/110580

(56) Entgegenhaltungen:
- DE-A1- 102018 121 496
- DE-A1- 102018 121 513
- US-A1- 2013 325 059
- US-A1- 2021 196 906
- US-B2- 7 021 561

## Beschreibung

Die Erfindung betrifft ein medizinisches Instrument zum multidirektionalen Verdüsen eines Fluids in einen Hohlraum eines Körpers und ein Werkzeug dafür.

Aus dem Stand der Technik sind Werkzeuge für medizinische Instrumente bekannt, die dafür ausgebildet sind, Fluide, insbesondere Fluide mit therapeutischen Substanzen, in einen Hohlraum eines Körpers zu bringen. Die WO 2012/163 346 A1 beschreibt ein Trokarsystem mit einem Werkzeug, das an seinem distalen Ende eine Düse aufweist, wobei das distale Ende in eine Körperhöhle hineinragen kann. Mit dieser Düse kann ein therapeutisches Fluid in einem Pneumoperitoneum versprüht werden.

Die Sprührichtung dieser Düse weist allerdings nur in eine Richtung, so dass das Fluid nicht multidirektional innerhalb des Bauchraums und damit gleichmäßig in alle Richtungen versprüht werden kann.

Werkzeuge mit mehreren Düsen sind aus der DE 10 2018 121 513 A1 bekannt, wobei dort der Werkzeugkopf drehbar in dem Schaft des Werkzeugs gelagert ist. Die Düsenkörper sind in dem kugelförmigen Werkzeugkopf in vorbestimmten Winkeln zueinander angeordnet so dass die Düsenöffnungen gleichmäßig über die Oberfläche des Werkzeugkopfes verteilt sind. Ein weiteres relevantes Dokument aus dem Stand der Technik ist DE 10 2018 121496 A1.

Ausgehend von diesem Stand der Technik ist es Aufgabe der vorliegenden Erfindung, ein verbessertes Werkzeug für ein medizinisches Instrument zum multidirektionalen Versprühen bzw. Verdüsen eines Fluid in einen Hohlraum eines Körpers bereitzustellen, der eine sichere Bedienung ermöglicht.

Diese Aufgabe wird durch ein Werkzeug mit den Merkmalen des Anspruchs 1 gelöst.

Die weitere Aufgabe, ein medizinisches Instrument bereitzustellen, das zum multidirektionalen Versprühen bzw. Verdüsen eines Fluid in einen Hohlraum eines Körpers ausgebildet ist und das sichere Bedienen des damit verbundenen Werkzeugs ermöglicht, wird durch das medizinische Instrument mit den Merkmalen des Anspruchs 10 gelöst.

Weiterbildungen des Werkzeugs und des medizinischen Instruments sind in den Unteransprüchen ausgeführt.

Gemäß einer ersten Ausführungsform eines erfindungsgemäßen Werkzeugs, das mit einem medizinischen Instrument eingesetzt wird, um ein Fluid in einen Hohlraum eines Körpers multidirektionalen verdüsen zu können, weist das Werkzeug einen Schaft mit einem Lumen auf. An dem distalen Ende des Werkzeuges ist ein Düsenkopf angeordnet, der zwei oder mehr seitliche Düsen und eine distale Düse hat; die distale Düse ist an einer distalen Stirnseite des Düsenkopfes angeordnet. Jede Düse hat eine Düsenöffnung, die über einen Verteilerzylinder fluidisch mit dem Lumen verbunden ist. Dabei ist eine Kappe um den Düsenkopf lösbar angeordnet, die für jede der Düsen eine über deren Düsenöffnung positionierte Durchgangsöffnung aufweist, die dazu ausgebildet ist, einen Strahl des zu verdüsenden Fluids austreten zu lassen. Die Durchgangsöffnungen der Kappe weisen jeweils einen Durchmesser auf, der kleiner ist als ein Durchmesser der jeweiligen unter der Durchgangsöffnung positionierten Düse.

Unter "Hohlraum" eines Körpers, in den ein Fluid einzubringen ist, kann hierin jede Art von Hohlraum in einem Menschen oder eines Tieres verstanden werden. Ein solcher Hohlraum kann bspw. die Bauchhöhle sein. Als Fluid kommt jede fluide therapeutische Substanz, wie ein Medikament, Medikament in Lösung oder auch eine simple Spüllösung in Frage, sofern sie durch Versprühen und folglich "Verdüsen" im Sinne von Ausgeben mittels Düse in den Hohlraum des Körpers eingebracht werden kann. "Verdüsen" meint hierin also das Aufteilen des in dem Werkzeug strömenden Fluids in eine große Zahl von Einzeltröpfchen durch Pressen durch eine Düse und ist gleichzusetzen mit Versprühen oder über eine Düse ausbringen oder - je nach eingesetzter Düse bzw. Düsenöffnung - vernebeln. Unter "Düse" wird ein Bauteil verstanden, das einen Düsenkörper (bevorzugt zylindrisch, aber nicht darauf beschränkt) aufweist, in dem zumindest eine Durchgangsbohrung oder -öffnung für ein zu verdüsendes Fluid ausgebildet ist.

Das in den Hohlraum einzubringende Fluid wird erfindungsgemäß vorteilhaft durch die distale Düse und die seitlichen Düsen multidirektional verdüst. Die Kappe bietet dabei vorteilhaft einen Rückhalt für die Düsenkörper der Düsen und eine zusätzliche Sicherheit, da das Fluid in dem Werkzeuglumen einen hohen Druck erzeugen kann und auf die Düsenkörper der Düsen sich derart auswirken kann, dass die Düsen aus ihrer Verankerung im Düsenkopf herausgesprengt werden und bei Einsatz in einem Patienten diesen verletzen können. Die Kappe verhindert dies gerade dadurch, dass sie den Düsenkörper vollständig umgibt und die Düsenkörper durch ihre größeren Abmessungen im Vergleich zu den der Durchgangsöffnungen der Kappe an der Wandung festgehalten werden.

Vorteilhaft bietet damit die erfindungsgemäße Anordnung der Kappe eine Lösung gegenüber dem Stand der Technik, bei dem die Düsenkörper ungesichert im Werkzeugkopf angeordnet sind und eben deshalb durch das zu versprühende Fluid, das innerhalb des Werkzeugs einen hohen Druck erzeugt, aus dem Werkzeugkopf herausgesprengt werden und einen Patienten oder den Bediener des Werkzeugs verletzen können.

Die Durchgangsöffnung mündet in eine Ausnehmung in der Mantelfläche der Kappe, die sich in Bezug auf die Mantelfläche der Kappe konisch von innen nach außen erweitert. Diese Ausnehmung dient zur Führung des aus den Düsen austretenden Fluids. Die Durchgangsöffnungen können in einer weiteren Ausführungsform des erfindungsgemäßen Werkzeugs auch kleiner sein als die Austrittsöffnungen der Düsenkörper der Düsen. Wenn die Durchgangsöffnungen kleiner sind als die Austrittsöffnungen, können stärkere Kräfte, die in den Düsenkörpern der Düsen durch das komprimierte und mit hoher Geschwindigkeit durchströmende Fluid entstehen, geleitet, abgefangen oder geschickt verteilt werden, als wenn die Durchgangsöffnungen und die Austrittsöffnungen gleich groß sind, und damit die Rückhaltefunktion noch verstärken.

Der Düsenkopf weist nach einer weiteren Ausführungsform des erfindungsgemäßen Werkzeugs drei, vier oder mehr seitliche Düsen auf. Die Düsen sind in radialer Richtung gerichtet, so dass das zu verdüsende Fluid in einem rechten Winkel zu einer WerkzeugLängsrichtung austreten kann. Das zu verdüsende Fluid kann hiermit besonders gut multidirektional in einen Hohlraum eines Körpers versprüht werden. Bevorzugte Sprühwinkel der Düsen liegen in einem Winkelbereich von 50° bis 120°, besonders bevorzugt wird ein Winkel von 90° aufgespannt. Damit kann für unterschiedliche Anwendungen ein anderer Winkelbereich bzw. multidirektionales Versprühen in vorbestimmte Richtungen erzielt werden.

Ferner können nach noch einer weiteren Ausführungsform des erfindungsgemäßen Werkzeugs die seitlichen Düsen in Bezug zu einer Mittelachse des Schafts in axialer Richtung äquidistant voneinander angeordnet sein. Vorteilhaft für gleichmäßiges Ausbringen des Fluids können die Winkel, um die die Düsen in Bezug zu der Mittelachse voneinander beabstandet sind, bei drei Düsen bei 120°, bei vier Düsen entsprechend bei 90° liegen. Es sind auch andere, nicht äquidistante Winkelkombinationen möglich, bspw. wenn die Verdüsung des Fluids in eine bestimmte Richtung, die durchaus einen großen Winkel umfassen kann, gerichtet erfolgen soll. Die Düsen liegen entlang der Mittelachse des Schafts auf der gleichen Ebene, können aber auch auf unterschiedlichen Ebenen abgestuft vorliegen, so dass die seitlichen Düsen spiralförmig entlang der Längsausdehnung des Düsenkopfes angeordnet sein können.

Nach noch einer weiteren Ausführungsform des erfindungsgemäßen Werkzeugs hat der Düsenkopf einen distalen Abschnitt, der eine erste Befestigungsvorrichtung aufweist. Die Kappe hat entsprechend eine zweite Befestigungsvorrichtung, die mit der ersten Befestigungsvorrichtung in Eingriff gebracht werden kann, um eben die Kappe an dem Düsenkopf zu befestigen.

Es ist besonders vorteilhaft, wenn sich der Düsenkopf an dem distalen Abschnitt über eine stufenartige Schulter verjüngt, an dem die erste Befestigungsvorrichtung angeordnet ist. Die Anordnung der Befestigungsvorrichtung an dem distalen Abschnitt bietet den zusätzlichen Vorteil, dass die Kappe fest auf dem Düsenkopf angebracht und gehalten werden kann, wodurch die Düsen noch besser davor geschützt werden können, herausgesprengt zu werden.

Die erste Befestigungsvorrichtung kann ein Außengewinde an dem Düsenkopf sein, dann ist die zweite Befestigungsvorrichtung ein Innengewinde an der Kappe, die kann nach Montage des Düsenkopfes an dem Schaft einfach auf den Düsenkopf aufgedreht werden. Die Gewindeverbindung kann ferner noch abgedichtet oder verklebt werden. Die Schulter zwischen Düsenkopf und distalem Abschnitt bietet eine weitere Klebe- bzw. Abdichtfläche und kann als Anschlag für das Gegengewinde der Kappe dienen.

Alternativ kann die Kappe auch auf den Düsenkopf aufgeklebt werden oder der proximale Rand der Kappe kann mit dem Düsenkopf verschweißt oder verpresst werden. Ferner sind weitere Alternativen möglich wie ein Bajonett-Verschluss oder eine Nut-Feder-Verbindung.

Der Düsenkopf ist zylindrisch. An seinem dem Schaft zugewandten Ende verjüngt sich der Düsenkopf über eine Schulter und bildet einen hohlzylindrischen Aufnahmeabschnitt aus. Dieser hohlzylindrische Aufnahmeabschnitt erstreckt sich im Montagezustand in den Schaft. In dem Lumen ist ein Stift aufgenommen. Dabei ist zwischen dem Außenumfang des Stiftes und dem Innenumfang des Schaftes ein Spalt gebildet, der einen Fluidpfad für das Fluid bereitstellt. Der Stift erstreckt sich in proximaler Richtung bis in die Nähe eines Anschlussstückes des Schafts und schafft so einen durchgängigen Spalt, der für eine gleichmäßige Fluidströmung durch den Schaft bis zum Düsenkopf sorgt.

An seinem dem Düsenkopf zugewandten Ende weist der Stift einen Abschnitt mit einem verjüngten Außenumfang auf. Während der hohlzylindrische Aufnahmeabschnitt des Düsenkopfes sich in den Schaft erstreckt, umgibt der hohlzylindrische Aufnahmeabschnitt den Abschnitt mit dem verjüngten Außenumfang des Stiftes. Der Stift erstreckt sich somit in den Düsenkopf hinein. Die Aufnahmeabschnitte der Bauteile Stift und Düsenkopf können Befestigungen aufweisen. Dies können Gewinde oder ineinandergreifende Hinterschnitte sein. Die Bauteile können auch miteinander verpresst oder verklebt sein.

Nach noch einer weiteren Ausführungsform des erfindungsgemäßen Werkzeugs weist der Stift nahe seinem Abschnitt mit dem verjüngten Außenumfang eine radiale Durchgangsbohrung auf. "Nahe" meint hierin, dass die radiale Durchgangsbohrung benachbart zu dem Abschnitt mit dem verjüngten Außenumfang bzw. in einer Distanz dazu angeordnet ist, die klein ist im Vergleich zu der Gesamtlänge des Stifts. Ferner hat der Stift an dem Abschnitt mit dem verjüngten Außenumfang eine zentralaxiale Bohrung, deren eines Ende in den Düsenkopf und deren anderes Ende in die radiale Durchgangsbohrung mündet. Hiermit wird die fluidische Verbindung zwischen dem Lumen und dem Verteilerzylinder in dem Düsenkopf ermöglicht.

Die Düsen können nach noch einer weiteren Ausführungsform des erfindungsgemäßen Werkzeugs zylindrisch ausgebildet sein. Dies lässt es zu, einen konstruktiv einfachen Düsenkopf zu konstruieren, der ebenso zylindrische Ausnehmungen für die Düsen aufweist, in die die Düsen eingeklebt, eingepresst, eingeschweißt oder auch eingedreht werden können.

Ferner weist der Schaft nach noch einer weiteren Ausführungsform des erfindungsgemäßen Werkzeugs an seinem proximalen Ende einen Anschluss zur fluidischen Verbindung des Lumens mit einer Fluidquelle auf, wobei der Anschluss ein Luer-Lock-Anschluss sein kann. Damit kann das Werkzeug an alle möglichen Handgriffe bzw. Handhaben zur Verwendung des Werkzeugs angeschlossen werden. Alternativ kann die fluidische Verbindung durch einen Schlauch hergestellt werden, der direkt an dem proximalen Ende des Schafts befestigt ist.

Der Düsenkopf kann durch ein generatives Fertigungsverfahren hergestellt werden und passgenau auf den Schaft abgestimmt sein. Ein Außendurchmesser des Schafts kann in einem Bereich von 1 mm bis 30 mm liegen; 8 mm bis 10 mm sind bevorzugt. Diese Abmessung passt in bestehende Trokarsysteme hinein.

Die Erfindung bezieht sich ferner auf ein medizinisches Instrument zum Einbringen von Fluiden in einen Hohlraum eines Körpers. Das Instrument kann eine Handhabe, eine Fluidquelle sowie ein Werkzeug aufweisen, wobei das Werkzeug mit der Handhabe lösbar und mit der Fluidquelle fluidisch verbunden werden kann. Verwendet wird das erfindungsgemäße Werkzeug.

Nach einer weiteren Ausführungsform des medizinischen Instruments kann die Handhabe mit einem Trokar verbunden werden, so dass das Werkzeug mit dem Trokar innerhalb eines Trokarsystems verwendet werden kann. Ferner kann das medizinische Instrument mit der Fluidquelle über eine flexible Fluidleitung verbunden sein, wobei die Fluidquelle mit dem Werkzeug lösbar verbunden werden und das Werkzeug in den Trokar des medizinischen Instruments eingesteckt werden kann. Durch Betätigen von entsprechenden Betätigungselementen an der Handhabe bzw. an dem Trokar kann das Werkzeug mit der zu versprühenden Substanz beaufschlagt werden, indem eine fluidische Verbindung zwischen der Fluidquelle und dem Werkzeug hergestellt wird. Die fluide Substanz gelangt durch das Lumen zu dem Düsenkörper, wird durch die Mittelbohrung des Düsenkörpers zu dem Verteilerkonus geleitet und dort über die Zuführleitungen zu den Düsenöffnungen der Düsen. Die Substanz tritt aus den Düsenöffnungen aus und wird versprüht.

Weitere Ausführungsformen des Werkzeugs und des medizinischen Instruments sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung.

Dabei zeigt:
- **Fig. 1**: eine perspektivische Ansicht des erfindungsgemäßen medizinisches Instruments mit erfindungsgemäßem Werkzeug,
- **Fig. 2**: einen Längsschnitt durch das Werkzeug,
- **Fig. 3**: einen perspektivischer Teilschnitt durch den Düsenkopf mit Kappe,
- **Fig. 4**: einen Längsschnitt durch den Düsenkopf, und
- **Fig. 5**: einen Schnitt quer durch die in dem Düsenkopf gelagerten Düsen.

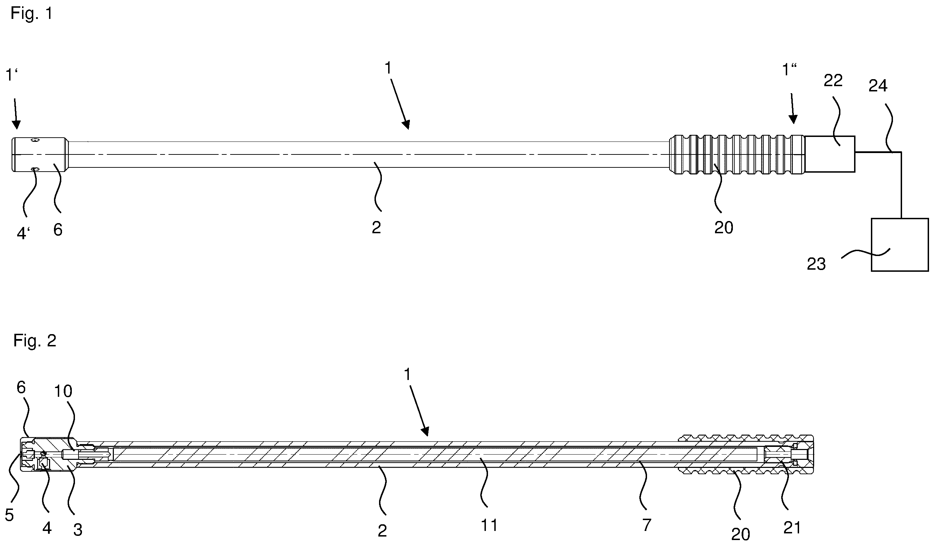

Das erfindungsgemäße medizinische Instrument weist ein Werkzeug 1 auf, das in **Fig. 1** einen Schaft 2 mit einem proximalen Ende 1" und einem distalen Ende 1' sowie einem innenliegenden Lumen 7 hat. Der Schaft 2 ist länglich und rohrfömig ausgebildet und hat in einer bevorzugten Ausführungsform einen Durchmesser von etwa 10 mm. Es sind Durchmesser in einem Bereich von 1 mm bis 30 mm möglich. Am proximalen Ende 1" ist ein Anschlussstück 20 angebracht, das einen sog. Luer-Lock-Anschluss 21 aufweist, der ein Standard-Anschluss bereitstellt, an den verschiedene Schläuche oder Flüssigkeitszugänge angeschlossen werden können. Der Luer-Lock-Anschluss 21 steht mit dem Lumen 7 fluidisch in Verbindung (siehe Längsschnittansicht in **Fig. 2****).**

Das Werkzeug 1 kann über das Anschlussstück 20 mit einer Handhabe 22 und einer Fluidquelle 23 verbunden werden, wie **Fig. 1** zeigt. Die Fluidquelle 23 ist über eine Fluidleitung 24 fluidisch mit der Handhabe 22 und damit mit dem Werkzeug 1 verbunden. Ferner kann das Werkzeug 1 auch zusammen mit einem Trokarsystem im Rahmen der minimalinvasiven Chirurgie verwendet werden (figurativ nicht dargestellt).

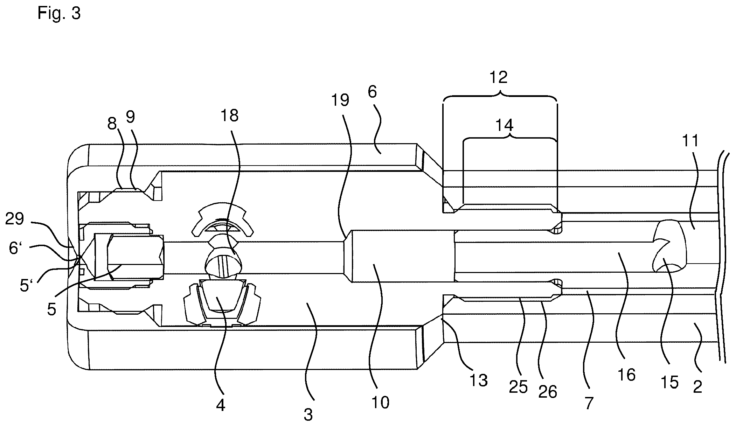

**Fig. 2** bis **5** zeigen das Werkzeug 1 in einzelnen Details.

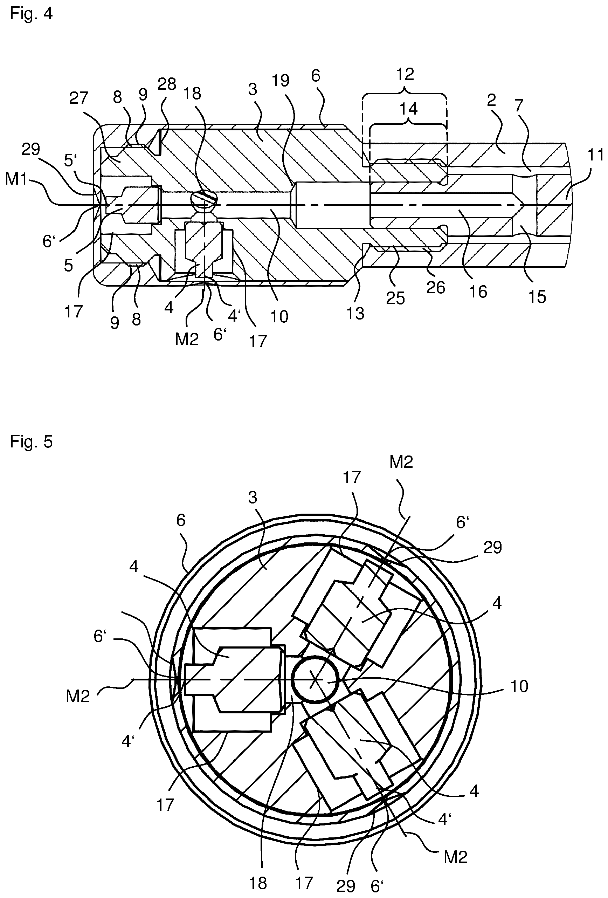

An dem distalen Ende 1' des Werkzeuges 1 ist ein zylindrischer Düsenkopf 3 angeordnet, der an seinem dem Schaft 2 zugewandten Ende eine Schulter 13 aufweist, über die sich der Düsenkopf 3 verjüngt und einen hohlzylindrischen Aufnahmeabschnitt 12 ausbildet. Mit diesem Aufnahmeabschnitt 12 ist der Düsenkopf in dem Schaft 2 eingesetzt. Wie **Fig. 4** zeigt, hat der Aufnahmeabschnitt 12 auf seiner Mantelfläche ein Außengewinde 25, das mit einem Innengewinde 26 an der Innenwandung des Schafts 2 in Eingriff steht. Ein Abschnitt eines Stifts 11, der in dem Lumen 7 aufgenommen ist und sich von dem Düsenkopf 3 bis kurz vor dem Anschlussstück 20 innerhalb des Schafts 2 erstreckt, ist in den Aufnahmeabschnitt 12 eingesteckt. Zwischen einem Außenumfang des Stiftes 11 und einem Innenumfang des Schaftes 2 ist ein Spalt gebildet, durch den ein Fluid strömen kann, wie **Fig. 2 bis 4** zeigen. An seinem dem Düsenkopf 3 zugewandten Ende weist der Stift 11 einen Abschnitt 14 mit einem verjüngten Außenumfang auf, mit dem der Stift 11 in den hohlzylindrischen Aufnahmeabschnitt 12 des Düsenkopfes 3 eingesteckt ist. Der Abschnitt 14 erstreckt sich bis kurz vor der Schulter 13 und stellt sicher, dass ein durchgängiger Fluidpfad für das zu verdüsende Fluid in dem Schaft 2 gebildet wird. Damit das Fluid aus dem Spalt zwischen Stift 11 und Innenwandung des Schafts 2 weitergeführt wird, weist der Stift 11 benachbart zu seinem Abschnitt 14 mit dem verjüngten Außenumfang eine radiale Durchgangsbohrung 15 auf, diese steht mit dem Spalt in dem Lumen 7 fluidisch in Verbindung. Um den Düsenkopf 3 fluidisch zu verbinden, hat der Abschnitt 14 mit dem verjüngten Außenumfang eine zentralaxiale Bohrung 16, deren eines Ende in den Düsenkopf 3 und deren anderes Ende in die radiale Durchgangsbohrung 15 mündet.

In dem Düsenkopf 3 ist ein zylindrischer Verteilerzylinder 10 eingebracht, der sich ausgehend von dem Aufnahmeabschnitt 12 bis zu der distalen Stirnseite des Düsenkopfes 3 erstreckt, wobei der Verteilerzylinder 10 sich an einer Stufe 19 in Richtung der distalen Stirnseite des Düsenkopfes 3 verjüngt. Von dem Verteilerzylinder 10 erstrecken sich Bohrungen 18 zum Einen in einer Ebene, die durch Mittelachsen M2 aufgespannt wird, radial nach außen und zum Anderen distal zur Stirnseite, und münden in zylindrische Ausnehmungen 17, in denen zylindrische Düsen 4, 5 angeordnet sind. Insgesamt weist der Düsenkopf 3 drei seitliche Düsen 4 und eine distale Düse 5 auf, die an der distalen Stirnseite des Düsenkopfes 3 (entsprechend dem distalen Ende des Werkzeuges 1') angeordnet ist. Die seitlichen Düsen 4 sind in Bezug zu einer Mittelachse M1 des Schafts 2 in axialer Richtung äquidistant voneinander beabstandet. Nach der Schnittzeichnung der **Fig. 5** schließen die Mittelachsen M2 der seitlichen Düsen 4 zueinander jeweils einen Winkel von 120° ein. Jede Düse 4, 5 hat eine Düsenöffnung 4', 5', die über den Verteilerzylinder 10 und den Bohrungen 15, 16 in dem Stift 11 fluidisch mit dem Lumen 7 verbunden ist. Die Ausnehmungen 17 sind so bemessen, dass die Düsenkörper der Düsen 4, 5 vollständig aufgenommen werden können. Sie weisen jeweils eine Abstufung auf, in der die Düsenkörper eingesetzt und fest montiert sind, wie in Fig. 4 gezeigt. Das "feste Montieren" kann durch Einkleben, Verschrauben oder Einklemmen erfolgen.

Um den Düsenkopf 3 ist eine Kappe 6 lösbar angeordnet. Die Kappe 6 weist für jede der Düsen 4, 5 eine über deren Düsenöffnung 4', 5' positionierte Durchgangsöffnung 6' auf. Diese in der Kappe 6 eingebrachten Durchgangsöffnungen 6' lassen einen Strahl des zu verdüsenden Fluids austreten. Die Abmessungen der Durchgangsöffnungen 6' der Kappe 6 haben jeweils einen Durchmesser, der kleiner ist als ein Durchmesser der jeweiligen unter der Durchgangsöffnung 6' positionierten Düse 4, 5. Jede Durchgangsöffnung 6' erweitert sich in eine konische Ausnehmung 29, um das aus den Düsen 4, 5 austretende Fluid zu führen. Der Durchmesser der Ausnehmung 29 ist, wie auch die Durchgangsöffnung 6', kleiner als der Durchmesser des Düsenkörpers der darunter angeordneten Düse 4, 5. Die konische Ausnehmung 29 verteilt die Kräfte, die durch das hochkomprimierte und durch die Durchgangsöffnung 6' austretende Fluid auf die Düse 4, 5 wirken. Um die Kappe 6 mit dem Düsenkörper zu verbinden, weist der Düsenkopf 3 einen distalen Abschnitt 27 an seiner Mantelfläche auf, der ein Außengewinde 8 hat, das als eine erste Befestigungsvorrichtung dient. Der Düsenkopf 3 verjüngt sich an dem distalen Abschnitt 27 über eine Stufe 28. Das Außengewinde 8 ist an einem Abschnitt des distalen Abschnitts 27 ausgebildet. Die Kappe 6 hat hierzu in einem zu dem distalen Abschnitt des Düsenkopfes 3 korrespondierenden Abschnitt ein Innengewinde 9, das als Gegengewinde zu dem Außengewinde 8 ausgebildet ist und als zweite Befestigungsvorrichtung dient. Damit kann die Kappe 6 fest aufgeschraubt werden. Die Stufe 28 am Düsenkopf 3 bildet eine weitere Fläche für eine mögliche Abdichtung oder als Anschlag für das Gegengewinde 9 der Kappe.

### BEZUGSZEICHENLISTE

- 1: Werkzeug
- 1': distales Ende Werkzeug
- 1": proximales Ende Werkzeug
- 2: Schaft
- 3: Düsenkopf
- 4: seitliche Düsen
- 4': Düsenöffnung seitliche Düsen
- 5: distale Düse
- 5': Düsenöffnung distale Düse
- 6: Kappe
- 6': Durchgangsöffnungen Kappe
- 7: Lumen
- 8: Außengewinde
- 9: Innengewinde
- 10: Verteilerzylinder
- 11: Stift
- 12: hohlzylindrische Aufnahmeabschnitt
- 13: Schulter
- 14: Abschnitt mit einem verjüngten Außenumfang
- 15: radiale Durchgangsbohrung
- 16: zentralaxiale Bohrung
- 17: Ausnehmung für Düsenkörper
- 18: radiale Bohrungen
- 19: Stufe Verteilerzylinder
- 20: Anschlussstück
- 21: Luer-Lock-Anschluss
- 22: Handhabe
- 23: Fluidquelle
- 24: Fluidleitungen
- 25: Außengewinde
- 26: Innengewinde
- 27: Distaler Abschnitt
- 28: Stufe
- 29: Ausnehmung Kappe
- M1: Mittelachse Schaft
- M2: Mittelachse Düsen

## Patentansprüche

1. Werkzeug (1) für ein medizinisches Instrument zum multidirektionalen Verdüsen eines Fluids in einen Hohlraum eines Körpers, wobei das Werkzeug (1) einen Schaft (2) mit einem Lumen (7) aufweist, und
wobei an dem distalen Ende (1') des Werkzeuges (1) ein Düsenkopf (3) angeordnet ist, der zumindest zwei seitliche Düsen (4) und eine distale Düse (5), die an einer distalen Stirnseite (3') des Düsenkopfes (3) angeordnet ist, aufweist,
wobei jede Düse eine Düsenöffnung (4',5') aufweist, die über einen Verteilerzylinder (10) fluidisch mit dem Lumen (7) verbunden ist, und eine Kappe (6) um den Düsenkopf (3) lösbar angeordnet ist, die für jede der Düsen (4, 5) eine über deren Düsenöffnung (4',5') positionierte Durchgangsöffnung (6') aufweist, die dazu ausgebildet ist, einen Strahl des zu verdüsenden Fluids austreten zu lassen, wobei die Durchgangsöffnungen (6') der Kappe (6) jeweils einen Durchmesser aufweisen, der kleiner ist als ein Durchmesser der jeweiligen unter der Durchgangsöffnung (6') positionierten Düse (4, 5),
wobei der Düsenkopf (3) zylindrisch ist und sich an seinem dem Schaft (2) zugewandten Ende über eine Schulter (13) verjüngt und einen hohlzylindrischen Aufnahmeabschnitt (12) ausbildet, und
wobei in dem Lumen (7) ein Stift (11) aufgenommen ist, und zwischen einem Außenumfang des Stiftes und einem Innenumfang des Schaftes (2) ein Spalt bereitgestellt ist, der einen Fluidpfad für das Fluid bereitstellt,
wobei an seinem dem Düsenkopf (3) zugewandten Ende der Stift (11) einen Abschnitt (14) mit einem verjüngten Außenumfang aufweist und der hohlzylindrische Aufnahmeabschnitt (12) des Düsenkopfes (3) sich in den Schaft (2) erstreckt und zumindest einen Teil des Abschnitts (14) mit dem verjüngten Außenumfang des Stiftes (11) umgibt.

2. Werkzeug (1) nach Anspruch 1, wobei der Düsenkopf (3) drei, vier oder mehr seitliche Düsen (4) aufweist.

3. Werkzeug (1) nach Anspruch 1 oder 2, wobei die seitlichen Düsen (4) in Bezug zu einer Mittelachse (M1) des Schafts (2) in axialer Richtung äquidistant zueinander beabstandet sind.

4. Werkzeug (1) nach zumindest einem der Ansprüche 1 bis 3, wobei die Durchgangsöffnung (6') in eine Ausnehmung in der Mantelfläche der Kappe mündet, die sich in Bezug auf die Mantelfläche der Kappe konisch von innen nach außen erweitert.

5. Werkzeug (1) nach zumindest einem der Ansprüche 1 bis 4, wobei der Düsenkopf (3) einen distalen Abschnitt (27) aufweist, der eine erste Befestigungsvorrichtung aufweist,
und wobei die Kappe (6) eine zweite Befestigungsvorrichtung aufweist, die mit der ersten Befestigungsvorrichtung in Eingriff bringbar ist.

6. Werkzeug (1) nach Anspruch 5, wobei der Düsenkopf (3) sich an dem distalen Abschnitt (27) über eine Stufe (28) verjüngt und die erste Befestigungsvorrichtung an dem distalen Abschnitt (27) angeordnet ist.

7. Werkzeug (1) nach Anspruch 5 oder 6, wobei die Befestigungsvorrichtung ein Außengewinde (8) an dem Düsenkopf (3) und die zweite Befestigungsvorrichtung ein Innengewinde (9) an der Kappe (6) ist.

8. Werkzeug (1) nach Anspruch 1, wobei der Stift (11)
- nahe seinem Abschnitt (14) mit dem verjüngten Außenumfang eine radiale Durchgangsbohrung (15) aufweist,
- und an dem Abschnitt (14) mit dem verjüngten Außenumfang eine zentralaxiale Bohrung (16) aufweist,
deren eines Ende in den Düsenkopf (3) und deren anderes Ende in die radiale Durchgangsbohrung (15) mündet.

9. Werkzeug (1) nach zumindest einem der Ansprüche 1 bis 8, wobei die Düsen (4, 5) zylindrisch sind.

10. Medizinisches Instrument zum multidirektionalen Verdüsen eines Fluids in einen Hohlraum eines Körpers, mit einer Handhabe (22), einer Fluidquelle (23) sowie einem Werkzeug, das mit der Handhabe (22) lösbar und mit der Fluidquelle (23) fluidisch verbindbar ist, wobei das Werkzeug ein Werkzeug (1) nach zumindest einem der Ansprüche 1 bis 9 ist.

## Claims

1. A tool (1) for a medical instrument for the multidirectional atomizing of a fluid into a cavity of a body, wherein the tool (1) has a shaft (2) with a lumen (7), and
wherein a nozzle head (3), which has at least two lateral nozzles (4) and one distal nozzle (5), is arranged on the distal end (1') of the tool (1), said distal nozzle being arranged on a distal end face (3') of the nozzle head (3),
wherein each nozzle has a nozzle opening (4', 5'), which is fluidically connected to the lumen (7) via a distributor cylinder (10), and a cap (6) is releasably arranged around the nozzle head (3), which cap has, for each of the nozzles (4, 5), a through opening (6'), which is positioned over the nozzle opening (4', 5') thereof and which is formed to allow the discharge of a stream of the fluid to be atomized, wherein the through openings (6') of the cap (6) each have a diameter, which is smaller than a diameter of the respective nozzle (4, 5) positioned under the through opening (6'),
wherein the nozzle head (3) is cylindrical and tapers over a shoulder (13) on its end facing the shaft (2) and forms a hollow-cylindrical receiving section (12), and
wherein a pin (11) is received in the lumen (7), and a gap, which provides a fluid path for the fluid, is provided between an outer circumference of the pin and an inner circumference of the shaft (2),
wherein on its end facing the nozzle head (3), the pin (11) has a section (14) with a tapered outer circumference and the hollow-cylindrical receiving section (12) of the nozzle head (3) extends into the shaft (2) and surrounds at least a portion of the section (14) with the tapered outer circumference of the pin (11).

2. The tool (1) according to claim 1,
**wherein**
the nozzle head (3) has three, four or more lateral nozzles (4).

3. The tool (1) according to claim 1 or 2,
**wherein**
the lateral nozzles (4) are arranged equidistantly from one another in the axial direction with respect to a central axis (M1) of the shaft (2).

4. The tool (1) according to at least any one of claims 1 to 3,
**wherein**
the through opening (6') opens out into a recess in the jacket surface of the cap, which widens conically from the inside to the outside with respect to the jacket surface.

5. The tool (1) according to at least any one of claims 1 to 4,
**wherein**
the nozzle head (3) has a distal section (27), which has a first fastening device, and wherein the cap (6) has a second fastening device, which can be brought into engagement with the first fastening device.

6. The tool (1) according to claim 5,
**wherein**
the nozzle head (3) tapers on the distal section (27) over a step (28) and the first fastening device is arranged on the distal section (27).

7. The tool (1) according to claim 5 or 6,
**wherein**
the fastening device is an external thread (8) on the nozzle head (3) and the second fastening device is an internal thread (9) on the cap (6).

8. The tool (1) according to claim 1,
**wherein**
the pin (11)
- has a radial through bore (15) close to its section (14) with the tapered outer circumference,
- and has a central-axial bore (16) on the section (14) with the tapered outer circumference,
the one end of which opens out into the nozzle head (3) and the other end of which opens out into the radial through bore (15).

9. The tool (1) according to at least any one of claims 1 to 8,
**wherein**
the nozzles (4, 5) are cylindrical.

10. A medical instrument for the multidirectional atomizing of a fluid into a cavity of a body,
with a handle (22), a fluid source (23) as well as a tool, which can be releasably connected to the handle (22) and can be fluidically connected to the fluid source (23),
**wherein**
the tool is a tool (1) according to at least any one of claims 1 to 9.

## Revendications

1. Outil (1) pour un instrument médical destiné à la pulvérisation multidirectionnelle d'un fluide dans une cavité corporelle, l'outil (1) comportant une tige(2) dotée d'une lumière (7), et une tête de buse (3) étant disposée à l'extrémité distale (1') de l'outil (1), laquelle tête de buse comporte au moins deux buses latérales (4) et une buse distale (5) disposée sur une face frontale distale (3') de la tête de buse (3), chaque buse comportant une ouverture de buse (4', 5') reliée fluidiquement à la lumière (7) par un cylindre de distribution (10), et un capuchon (6) étant disposé de manière amovible autour de la tête de buse (3), lequel capuchon présente, pour chacune des buses (4, 5), une ouverture de passage (6') positionnée au-dessus de l'ouverture de buse (4', 5') correspondante, qui est conçue pour laisser sortir un jet du fluide à pulvériser, les ouvertures de passage (6') du capuchon (6) présentant chacune un diamètre inférieur à celui de la buse (4, 5) respective positionnée sous l'ouverture de passage (6'), la tête de buse (3) étant cylindrique, se rétrécissant à son extrémité tournée vers la tige (2) par un épaulement (13) et formant une section de réception cylindrique creuse (12), et une tige (11) étant logée dans la lumière (7) et un interstice étant prévu entre une périphérie extérieure de la tige et une périphérie intérieure de la tige (2), lequel interstice constitue un passage pour le fluide, la tige (11) présentant, à son extrémité tournée vers la tête de buse (3), une section (14) munie d'une périphérie extérieure rétrécie, et la section de réception cylindrique creuse (12) de la tête de buse (3) s'étendant dans la tige (2) et entourant au moins une partie de la section (14) munie de la périphérie extérieure rétrécie de la tige (11).

2. Outil (1) selon la revendication 1,
**dans lequel**
la tête de buse (3) comporte trois, quatre ou plus de buses latérales (4).

3. Outil (1) selon la revendication 1 ou 2,
**dans lequel**
les buses latérales (4) sont espacées les unes des autres de façon équidistante dans la direction axiale par rapport à un axe central (M1) de la tige (2).

4. Outil (1) selon au moins l'une des revendications 1 à 3,
**dans lequel**
l'ouverture de passage (6') débouche dans un évidement de la surface latérale du capuchon, qui s'élargit de façon conique de l'intérieur vers l'extérieur par rapport à la surface latérale du capuchon.

5. Outil (1) selon au moins l'une des revendications 1 à 4,
**dans lequel**
la tête de buse (3) comporte une section distale (27) qui comporte un premier dispositif de fixation,
et **dans** lequel le capuchon (6) comporte un deuxième dispositif de fixation pouvant être mis en prise avec le premier dispositif de fixation.

6. Outil (1) selon la revendication 5,
**dans lequel**
la tête de buse (3) se rétrécit au niveau de la section distale (27) par un gradin (28) et le premier dispositif de fixation est disposé sur la section distale (27).

7. Outil (1) selon la revendication 5 ou 6,
**dans lequel**
le dispositif de fixation est un filetage extérieur (8) sur la tête de buse (3) et le deuxième dispositif de fixation est un filetage intérieur (9) sur le capuchon (6).

8. Outil (1) selon la revendication 1,
**dans lequel** la tige (11)
- présente, à proximité de sa section (14) munie d'une périphérie extérieure rétrécie, un alésage radial traversant (15),
- et, au niveau de la section (14) munie d'une périphérie extérieure rétrécie, un alésage axial central (16),
dont une extrémité débouche dans la tête de buse (3) et dont l'autre extrémité débouche dans l'alésage radial traversant (15).

9. Outil (1) selon au moins l'une des revendications 1 à 8,
**dans lequel**
les buses (4, 5) sont cylindriques.

10. Instrument médical destiné à la pulvérisation multidirectionnelle d'un fluide dans une cavité corporelle,
comprenant une poignée (22), une source de fluide (23) ainsi qu'un outil qui peut être fixé de façon amovible à la poignée (22) et relié fluidiquement à la source de fluide (23),
**dans lequel**
l'outil est un outil (1) selon au moins l'une des revendications 1 à 9.
